# EUROPEAN PATENT SPECIFICATION

(11) **EP 3 002 275 B1**
(45) Date of publication and mention of the grant of the patent: **14.07.2021**
(21) Application number: 14003393.7
(22) Date of filing: 01.10.2014
(51) Int. Cl.: C07C 231/02, C07C 231/12, C07C 233/36, C07C 233/38, C11D 1/90, C11D 1/94

(54) **PROCESS FOR PREPARING A CONCENTRATED, NON-GELLING, AQUEOUS SOLUTION OF BETAINE**
VERFAHREN ZUR HERSTELLUNG KONZENTRIERTER, NICHT GELBILDENDER, WÄSSRIGER LÖSUNGEN EINES BETAINS
PROCESSUS DE PRÉPARATION DE SOLUTIONS AQUEUSES CONCENTRÉES NON-GÉLIFIANT DE BÉTAINE

(43) Date of publication of application: 06.04.2016
(73) Proprietor: Hayat Kimya Sanayi Anonim Sirketi, 34662 Altunizade, Uskudar/Istanbul (TR)
(72) Inventor: Acikalin, Korkut, Basiskele/Kocaeli (TR); Isiklar, Ahmet Nezir, Basiskele/Kocaeli (TR); Koc, Fikret, Basiskele/Kocaeli (TR)
(74) Representative: Dericioglu, E. Korhan

(56) References cited:
- EP-A1- 0 677 509
- EP-A2- 0 353 580
- EP-A2- 0 647 613
- BE-A0- 1 017 779
- DE-C1- 4 207 386
- VESNA KOSTIK ET AL: "FATTY ACID COMPOSITION OF EDIBLE OILS AND FATS", JOURNAL OF HYGIENIC ENGINEERING AND DESIGN, vol. 4, 1 January 2013 (2013-01-01), pages 112-116, XP055202426,
- None

## Description

### FIELD OF THE INVENTION

The present invention relates to a process for surfactant preparation. Particularly, the present invention relates to a process for the preparation of concentrated, flowable and pumpable low-viscosity aqueous solutions of betaine with a betaine content of 35% by weight of the solution. The resultant betaine solutions produced according to additive-free process of the present invention have viscosity values ≤175 cP, and are pale yellow colour. The resultant betaine solutions produced according-to additive-free process of the present invention have low gel point and freezing point so that they are flowable and pumpable at low temperatures.

The process of the present invention does not involve the addition of any additional organic solvents, acids, micellar thickeners, fluidizers, surfactants, liquefying agents or such. Since the preferable process disclosed in the present invention does not involve the usage of any chemicals or materials during or before or after any part of the process, the present process can be adopted to any existing betaine production process easily and therefore, the cost of the betaine preparation process can be reduced by eliminating the use of additives.

The betaine solutions obtained by the present inventive process, thanks to their excellent dermal compatibility, can be used in the manufacture of products for cleaning purposes that require contact with the human skin such as liquid hand soaps, washing-up liquids, preferably in manual dishwashing detergents.

### BACKGROUND OF THE INVENTION

Betaines are amphoteric type surfactants, and exhibit an important place in personal care and detergent formulations due to their mildness, foaming characteristics and excellent cleaning properties. Their production can be achieved by two serial reactions which are generally named as "amidation" and "carboxymethylation (or quaternization)" reactions. Amidation reaction applied in the past involves the reaction of a fatty acid stock (fatty acids, methyl esters of fatty acids, or fatty acid glycerine esters) with dimethylaminopropyl amine (3-dimethylaminopropylamine, DMAPA) to yield amidoamine (intermediate product) and by-product (water, methanol or glycerine). Water and methanol are generally separated from the reaction medium by distillation, whereas glycerine is generally left in the product stream. The fatty acid stock used in amidation reactions are generally coconut fatty acids, palm oil fatty acids or palm kernel fatty acids. Typical fatty acid stock may comprise a fatty acid stock compounds with 6, 8, 10, 12, 14, 16, 18, 20, 22 and 24 carbon atoms. The weight percentage and saturated fraction of these fatty acid compounds may vary substantially depending on whether the raw fatty acid mixture is treated (distilled, hydrogenated, cut, uncut or combinations thereof) or not. In carboxymethylation reaction applied in the past, the product stream obtained from the amidation reaction is reacted with monochloroacetic acid in the presence of an alkaline salt or directly with the corresponding salt of this acid (i.e. sodium monochloroacetate - SMCA) in aqueous medium to produce betaine along with an unavoidable by-product alkaline chloride, which is usually sodium chloride.

It is well known that when the concentration of an aqueous surfactant solution increases, its viscosity also increases. High concentration values cause high viscosity values leading to difficulties in transportation of the surfactant solutions, and even can form pasty or gel form products which are impossible to transport. Betaine is such a surfactant, and cannot be mobilized in case its concentration in aqueous solution exceeds about 30% by weight. Thus, betaine solutions produced in an afore-mentioned method are sold at betaine concentrations in the range of 30% by weight.

Numerous attempts have been made to prepare betaine solutions that are as concentrated and as fluid as possible, for reducing the transportation and storage costs, and to obviate the need for preservatives.

For instance, EP 1659109 claims to produce solutions of betaines with a betaine content of at least 32 wt. % by adding one or more micellar thickeners to reaction mixture before or during the quaternization reaction where the micellar thickeners are mentioned as polyoxyethylene ethers and/or esters of a hydroxy fatty acid or of a hydroxy fatty acid glyceride.

EP 1289933 asserts that uncut cocoamidopropyl betaine composition having a betaine concentration at least 38 wt. % can be prepared by reducing the sodium chloride concentration of the carboxymethylation product to a value less than 15% based on the weight of surfactant and lowering cost factor.

EP 0937124 teaches us to prepare a concentrated aqueous betaine-type surfactant composition containing a dry residue of at least 40 wt. % by providing fluidizing additives before or after or during the quaternization reaction as 0.5-3.5% by weight of the surfactant solution. The fluidizing additives are determined as dimer fatty acids and phosphoric esters of fatty alcohols or polyethoxlylated fatty alcohols. Since later removal of these additives from the mixture is not possible, use of such reaction product mixtures are restricted only to uses where these additives are not intervening with targeted formulations. As such the use is restricted and disadvantageous.

EP 0958042 claims that compositions containing alkylamidopropylbetaine as 55-62 wt. % in the form of fluid solutions whose viscosity is less than 1000 mPa.s can be prepared provided that betaine/water/ethanol ternary compositions lies in specific ranges. EP 1008644 is also an application of the same applicant, and is similar to EP '042 relying on the betaine/water/ethanol ternary compositions to obtain concentrated alkylamidopropylbetaines. The use of ethanol in mixtures restricts its application due to flammability, light evaporation, skin incompability, noncompability with some formulations, hence rendering it disadvantageous.

EP 0992489 teaches us that betaines can be prepared by reacting castor oil and/or hydrogenated castor oil with diamines to form amidoamines, ethoxylating the resulting amidoamines with 1 to 50 ethylene oxide and reacting the resulted ethoxylated amidoamine with haloalkane acids or their salts. The betaines obtained by said method gives dry residue of 55.0% and a salt content of 5.9% by weight. Castor oil originated betaine products prepared according to above procedure are costly due to addition of ethoxy groups.

EP 0749412 states that low-viscosity water-containing concentrates of betaine surfactants with a solid content of 40-50 wt. % can be obtained by condensing the fatty acid amidoamines with halocarboxylic acid salts in the presence of liquefying agents, wherein the liquefying agents are determined as hydroxycarboxylic acids containing 2 to 6 carbon atoms.

EP 0730572 also states the use of liquefying agents, and additionally mentions about the polyol usage to obtain low-viscosity aqueous concentrates of betaine surfactants. Use of liquifying agents containing betaine products as such may be disadvantageous due to incompatibility issues by further usage and cost factors.

BE1017779 A0 discloses a process for preparing flowable solutions of alkylamidopropyl betaines. The synthesis comprises a step of amidation, in which fatty acid or a derivative thereof reacts with 3-dimethyl aminopropylamine and a second step of reaction of the amide with monochloroacetic acid in water. After completion of the reaction the reaction liquid is first cooled to a temperature of 70°C, then to room temperature.

All of the teachings to date include the use of an additional material or chemicals or additives such as micellar thickeners, fluidizers, acids, surfactants, viscosity modifiers, solvents, liquefying agents and the like during at least one part of the betaine preparation process to obtain concentrated betaine solutions whose betaine content is substantially higher than 30 % wt. Inclusion of additives to prepare concentrated betaine solutions are practically helpful but brings the disadvantages of cost increment and preclusion of the use of obtained betaine solution in every formulation since some additives may not be appropriate for every type of formulation or limits the usage flexibility of betaine solutions in some formulations.

It is therefore an object of the present invention to provide an additive-free process for the preparation of concentrated, flowable and pumpable low-viscosity aqueous solutions of betaine with a betaine content of ≤ 35% by weight.

### DESCRIPTION OF DISCLOSURE

In an effort to prepare high concentrated betaine solutions which are flowable and additive free the present inventor came to findings that flowable and pumpable low-viscosity betaine solutions with betaine content of 35% by weight can be reliably obtained without the use of any additives in any part of the betaine preparation process. According to the teaching of the present inventive process, this can be achieved by using the carefully and accurately selected fatty acid stock comprising 11-15 wt. % C₈ + C₁₀ fractions and less than 9% wt. of total unsaturated C₁₈ fractions as reactant in amidation part and providing the cooling rate applied after the completion of carboxymethylation below 0.5°C/min. To the best of our knowledge, said method was neither used commercially nor mentioned in any literature to date.

In accordance with the present invention concentrated (up to 35% by wt.) concentrated, low-viscosity, flowable and pumpable aqueous betaine solution with a betaine content of the general formula (I), 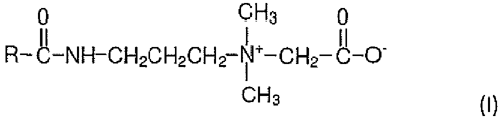 in which R is a mixture of C₈-C₁₈ alkyl of a fatty acid and C₈-C₁₈ unsaturated alkyl radical, can be prepared by the process comprising
(i) reacting a fatty acid stock comprising 11-15 wt. % C 8 +C10 fractions and less than 9% wt. of total unsaturated C18 fractions with 3-dimethylaminopropylamine in solvent free environment by removing of during the reaction formed water to yield amidoamine compound and
(ii) reacting in step (i) obtained amidoamine reaction mixture with sodium monochloroacetate or monochloroacetic acid in the presence of sodium hydroxide in an aqueous medium to yield a betaine solution
(iii) applying a gradual cooling process to the in step (ii) obtained betaine solution by use of a cooling medium
wherein in (iii) said gradual cooling process is carried out with a cooling rate which is below 0.5°C/ min and wherein in step (iii) said cooling process is carried out until the said betaine solution is cooled from 80-100°C, which is the temperature at which step (ii) takes place, to at least 70°C.

The term "betaine content" herein means the active betaine in betaine solution. It should not be confused with dry residue or dry content or solids content or dry matter terms which are generally 8-12 weight % higher than the betaine content.

The term "flowable" herein is used for being capable of flowing at room temperature and at low temperatures (-5°C - 20°C). Gel point and freezing point values of betaine solutions were used to determine the flowability at low temperatures (-5°C - 20°C).

The term "pumpable" herein is used for indicating the capability of being pumped at room temperature and at low temperatures (-5°C - 20°C). Gel point and freezing point values of betaine solutions are used to determine the pumpability at low temperatures (-5°C-20°C).

The term "low-viscosity" herein used for indicating the viscosity values of ≤ 175 cP at room temperature.

### BETAINE PRODUCTION PROCESS

Betaine production can be achieved by two serial reactions named as "amidation" and "carboxymethylation or quaternization".

### AMIDATION

The term "amidation reaction" is used interchangeably with "amidation step" within this description.

Amidation reaction is the first step in betaine production, and involves the reaction of fatty acid stock (fatty acids, methyl esters of fatty acids, or fatty acid glycerine esters) with dimethylaminopropyl amine (3-dimethylaminopropylamine, DMAPA) to yield amidoamine (intermediate product) and by-product (water, methanol or glycerine) according to the reaction (a):

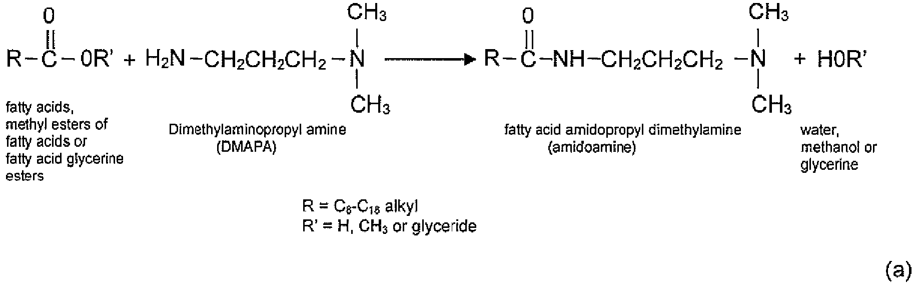

The preferred fatty acid stock of the present invention is fatty acids, and thus the amidation reaction can be shown by reaction (a-I):

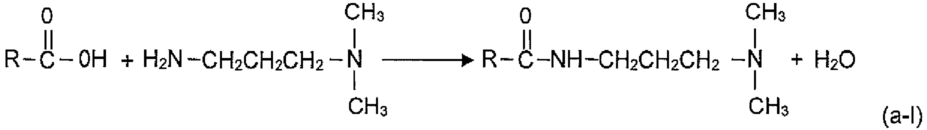

The present invention showed that the fatty acid stock used in the amidation step should contain less than 9% by weight of total unsaturated C₁₈ fractions and 11-15% by weight of C₈ + C₁₀ fractions in order to make possible to produce betaine solutions with a betaine content of 35% by weight without using any additives in any part of the production process. The fatty acid and DMAPA can be reacted in mole ratios of 1:1 to 1:1.1 preferably 1:1.03, more preferably 1:1.05 to achieve the completion of the reaction conversion. The amidation reaction can take place at 140-180°C, preferably at 150-170°C, more preferably at 155-165°C reaction temperature. The amidation reaction can take place for several hours (5,5-8 h) after having reached the desired reaction temperature, and is carried out until the acid value of sample taken from the amidation reaction medium is less than 12, preferably less than 10, more preferably less than 8 to ensure that final betaine solution includes less than 1% wt. of C₈-C₁₈ saturated and unsaturated free fatty acids. The by-product water formed during the amidation reaction is continuously removed from the reaction medium. The said by-product of the amidation reaction may include DMAPA about %30-40 by weight.

### CARBOXYMETHYLATION

The term "carboxymethylation reaction" is used interchangeably with "carboxymethylation step" .

Carboxymethylation (or quaternization) reaction is the second step in betaine production. At this step, the amidoamine obtained from the first step (amidation) is reacted with monochloroacetic acid in the presence of sodium hydroxide or directly with sodium monochloroacetate SMCA in aqueous medium to produce betaine along with the by-product sodium chloride. The latter reaction is as given below:

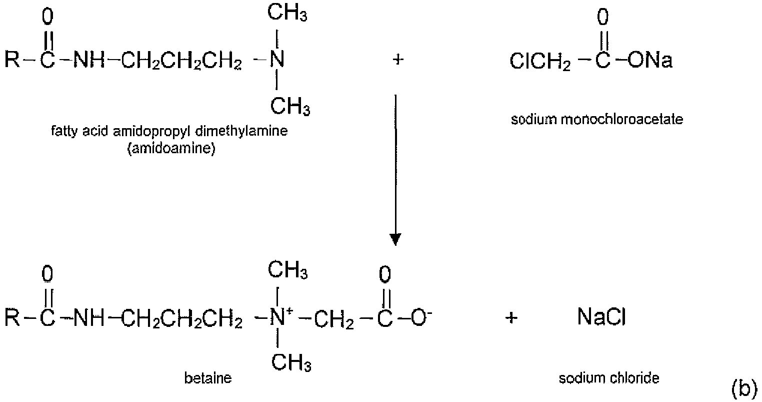

The amidoamine and sodium monochloroacetate can be reacted in mole ratios of 1:1 to 1:1.1,preferably 1:1.03, more preferably 1:1.05 to achieve the complete reaction conversion. The water addition at the beginning of the carboxymethylation reaction in order to provide aqueous medium should be tuned so that the resultant betaine solution can match the targeted betaine concentration by weight. The carboxymethylation reaction can take place at 80-100°C reaction temperature, preferably at 95-100°C. The heating rate applied to reach the desired reaction temperature can be 2-3°C/min. The carboxymethylation reaction can take place for several hours (6-7 h) after having reached the desired reaction temperature, and the completeness of reaction can be controlled by sodium chloride and/or betaine content analysis. The present invention surprisingly revealed that the cooling rate applied after the completion of carboxymethylation reaction has a crucial effect on the gel formation in resultant betaine solution. For obtaining a gelling free mixture the cooling rate at the end of the carboxymethylation is less than 0.5°C/min. The more preferable cooling rates (cooling rates below 0.5°C/min) result in 1-3 % weight more concentrated betaine solutions without gel formation when compared to the betaines obtained by fast cooling rates (cooling rates above 0.7°C/min) provided that all of the other production conditions (reaction temperature, reaction time, stoichiometric ratio of reactants, the water amount etc.) are kept same. The resultant betaine solution formed by the carboxymethylation reaction is cooled at least to 70°C since higher temperatures create higher temperature differences with the surrounding temperature, and thus may result in faster cooling rates than desired. It was also found that the surface of the reaction medium must be well-swept by the stirrer during the carboxymethylation step since, otherwise, a skin-like gel can be formed at the surface which prevents uniform cooling thus promotes gelation. Herein well-sweeping of the surface was maintained by immerging the stirrer into the reaction mixture so that at least some part of stirrer's blade was out of the reaction mixture while the other part of the same blade was in the reaction mixture. The preferred stirrer has multiple blades to provide well-swept surface and uniform mixing in the reaction mixture.

### ADDITIVE-FREE

Several attemps have been made in the past to obtain flowable betaine solutions as concentrated as possible. All of the teachings to date found in literature include the use of an additional material or chemical such as micellar thickeners, fluidizers, acids, surfactants, viscosity modifiers, solvents, liquefying agents etc. during at least one part (step) of the betaine preparation process to obtain concentrated betaine solutions whose betaine content is higher than 30 %wt. Inclusion of additives to obtain concentrated betaine solutions are practically helpful but brings the disadvantages of cost increment and preclusion of the use of obtained betaine solution in every formulation since some additives may not be appropriate for every type of formulation or limits the usage flexibility of betaine solutions in some formulations.

The present invention offers an additive-free process to obtain flowable, pumpable, low-viscosity aqueous solutions of betaines. Additive-free used herein means that no additional chemicals or other than the reactants of amidation and carboxymethylation steps are used in any stage of the process. In other words, the process subject to present invention includes only the usage of raw materials of amidation and carboxymethylation reactions which are fatty acid, DMAPA, amidoamine, sodium monocholoroacetate (or monochloroacetic acid at the presence of sodium hydroxide) and water.

The process of the present invention does not include the usage of any pH regulator (citric acid, hydrochloric acid or such) to adjust the pH of resultant betaine solution to the values of 4-6 which is a common and known method in most of the commercial production processes to maintain stability and low viscosity values.

The process of the present invention does not include any preservatives whose function is to provide resistance to growth of microorganisms in betaine solution. The betaine solutions (35% wt. betaine content) produced by the additive-free process of the present invention show adequate resistance to microorganism growth which was shown by the preservation efficacy test. The adequateness of preservation efficacy tests were attributed to high betaine content and low water content of the betaine solutions.

### OPTIONAL ADDITIVES -Glycerine and Trimethylamine (TMA)

Although the object of the present invention is to provide an additive-free process for the preparation of concentrated, flowable and pumpable low-viscosity aqueous solutions of betaine with a betaine content of 35% wt., some additives may be used optionally to gain some advantages as explained below.

The additives that can be used optionally are glycerine and trimethylamine (TMA).

Glycerine is an emollient that is often used in natural cosmetics and household cleaning - supplies to balance moisture levels and cleanse gently (skin friendly). Thus, its inclusion may be favourable for betaine solutions which are going to be used in manuel dishwashing detergent compositions. According to the present invention glycerine is optionally added before the carboxymethylation step, in other words, carboxymethylation step is carried out at the presence of glycerine. Glycerine addition is tuned so that the final betaine solution includes up to 4% wt. of glycerine. The study of the present invention shows that glycerine addition increases the viscosity of the final betaine solution when compared to the viscosity of betaine solution produced by the additive-free process.

TMA is the simplest tertiary amine which is very soluble in water. According to the present invention TMA is optionally added before the carboxymethylation step, in other words, carboxymethylation step is carried out at the presence of TMA. TMA addition is applied in the manner that 10:100 - 60:100 mole ratio of TMA:amidoamine is provided at the beginning of carboxymethylation reaction. It should be noted that at least stoichiometric amount of SMCA with regard to TMA should also be provided since TMA and SMCA reacts to yield trimethylglycine (TMG) at the carboxymethylation reaction conditions. The study of the present invention shows that TMA addition decreases the viscosity of the final betaine solution when compared to the viscosity of betaine solution produced by the additive-free process, probably due to the formation of low molecular weighted TMG during the carboxymethylation reaction.

### COOLING RATE

As used herein, "cooling rate" denotes the average cooling rate applied in cooling process of final betaine solution obtained from the completion of carboxymethylation reaction. Thus, the cooling process with the said cooling rate is the final stage of both carboxymethylation step and betaine production process.

Herein, said cooling rates were mentioned as "fast cooling rates" and "slow cooling rates". "Fast cooling rate" means that the cooling rates are ≥ 0.7°C/min whereas "slow cooling rates" are < 0.7°C/min.

In previous state of the technic the final betaine solution is cooled to ambient temperature at the end of the carboxymethylation step but no information was found about the applied cooling rates.

A fast cooling rate is applied in the past for convenience, fastness and cost effectiveness of the process.

The present inventor has surprisingly found that the cooling rate applied after the completion of carboxymethylation reaction has a crucial effect on the gel formation of final betaine solutions. The studies of the present invention showed that 1-3% wt. more concentrated betaine solutions without gel formation can be produced with slow cooling rates compared to the betaines produced by fast cooling rates provided that all of the other production conditions (reaction temperatures, reaction times, stoichiometric ratio of reactants, the water amount added etc.) in carboxymethylation reaction are kept the same. The cooling rate applied at the end of the carboxymethylation reaction is less than 0.5°C/min.

The resultant betaine solution formed by the carboxymethylation reaction should be cooled at least to 70°C, preferably at least to 65°C, more preferably at least to 60°C since higher temperatures create higher temperature differences with the surrounding temperature, and thus may result in faster cooling rates than desired. By cooling the betaine solutions at least to 70°C instead of ambient temperatures as stated in prior art, the cooling period can be shortened and an increase in production duration can be at least partly compensated.

In the embodiments of the present invention, the said cooling rate was provided by cooling the reaction flask with the ambient air or cooling the reaction flask with the hot silicone oil which is cooled by the ambient air or cooling the jacketed pilot reactor with circulating the hot heat-transfer oil in the jacket of the reactor while the hot-heat transfer oil was cooled by the ambient air.

But any known cooling process and cooling medium, preferably liquid cooling medium can be used to obtain targeted cooling rates,

### MIXTURE OF FATTY ACID

Fatty acid stock is the starting material of betaine production. It is firstly reacted with DMAPA to yield amidoamine in amidation step. The amidoamine obtained from the amidation step is then reacted with sodium monocholoroacetate (SMCA) in carboxymethylation step to produce betaine. Thus, the type of selected fatty acid stock greatly affects the properties of betaine. For instance, it is known that hardened (hydrogenated) fatty acid stock generally have better quality regarding to color and odor in betaine production (S. Herrwerth, H. Leidreiter, H.H. Wenk, M. Farwick, I. Ulrich-Brehm and B. Grüning, "Highly Concentrated Cocamidopropyl Betaine - The Latest Developments for Improved Sustainability and Enhanced Skin Care", Tenside Surf. Det. 45:304-308, 2008). It is also known that the carbon distribution strongly influences the detergency, foaming, and wetting properties of a surfactant. For a homologous series of surfactants, the maximum surface activity usually coincides with a C₁₂ chain length (T.F. Tadros, Applied Surfactants: Principles and Applications, Wiley-VCH, Weinheim, 2005). Thus, the fatty acid stock comprising abundant amounts of C₁₂ carbon chain are preferably used in betaine production as it was used in the present invention. The preferred amounts of C₁₂ proportion in fatty acid stock used in betaine production is at least %45, preferably at least 50% by weight.

The studies of the present invention indicated that the carbon distribution of fatty acid stock has also an important influence on the gelation of resultant betaine solution as well as the color, odor, detergency properties described above.

The present inventor has surprisingly found that fatty acid stock comprising less than 9% by weight of total unsaturated C₁₈ fractions and 11-15% by weight of C₈ + C₁₀ fractions together has resulted betaine solutions without any gelation at 35% betaine concentration by weight.

But the fatty acid stock which are used in previous state of the art in betain production did not satisfy the above conditions and showed gelation even at 32% betaine concentration by weight or had a high freezing point and/or gel point which causes immobilization of betaine solution at low temperatures.

Fatty acid stock types used for betaine production may be fatty acids, methyl esters of fatty acids, or the fats themselves (such as fatty acid glycerine esters). Independently from the fatty acid stock type, the fatty acid stock may include fatty acids having carbon distribution between C₆-C₂₄ including saturated and unsatured ones (See Table 1). The preferred carbon distribution of the fatty acid stock is C₈-C₁₈ since C₆ may cause odor problems and ≥C₂₀ may cause higher viscosity values in betaine solutions as it is known that the viscosities of the fatty acids and esters increases by an increase in the number of carbon atoms in the fatty acid chain (A.T. Gros, R.O. Feuge, "Surface and Interfacial Tensions, Viscosities, and other Physical Properties of some n-aliphatic acids and Their Methyl and Mthyl Esters", Journal of the American Oil Chemists Society 29(8):313-317, 1952).

The studies of the present invention showed that C₈ and C₁₀ fractions must be available in the fatty acid stock 11-15% by weight to reach the targets of the present invention. The preferred C₈:C₁₀ ratio by weight is between 40:60 and 60:40, more preferably 45:55 and 55:45. The studies of the present invention also clearly indicates that an unsatured C₁₈ fraction (i.e. C_{18:1}, C_{18:2}) must be present in the fatty acid stock, and the amount of total unsaturated C₁₈ fraction in the fatty acid stock must be less than 9% wt. to accomplish the purpose of the present invention. The preferred C_{18:1}:C_{18:2} ratio by weight is between 75:25 - 95:05, more preferably between 85:15 - 90:10. The preferred amounts of C₁₄, C₁₆ and C₁₈ fractions of the fatty acid stock are 15-21%, 5-15%, and ≤5%, more preferably 17-19%, 6-9% and ≤2%, respectively.

**Table 1. Symbol, Systematic Name and Trival Name of Fatty Acids**

| Symbol | Systematic name | Trivial name | Symbol | Systematic name | Trivial name |
|---|---|---|---|---|---|
| Saturated fatty acids | | | Unsaturated fatty acids | | |
| C₆ | hexanoic acid | caproic acid | C_{16:1} | 9-hexadecenoic | palmitoleic |
| C₈ | octanoic acid | caprylic acic | C_{18:1} | 9-octadecenoic | oleic |
| C₁₀ | decanoic | capric | C_{18:2} | 9,12-octadecadienoic | linoleic |
| C₁₂ | dodecanoic | lauric | C_{18:3} | 9,12,15-octadecatrienoic | linolenic |
| C₁₄ | tetradecanoic | myristic | C_{20:1} | 9-eicosenoic | gadoleic |
| C₁₆ | hexadecanoic | palmitic | C_{20:4} | 5,8,11,14-eicosatetraenoic | arachidonic |
| C₁₈ | octadecanoic | stearic | C_{22:1} | 13-docosenoic | erucic |
| C₂₀ | eicosanoic | arachidic | | | |
| C₂₂ | docosanoic | behenic | | | |
| C₂₄ | tetracosanoic | lignoceric | | | |

The fatty acid stock used in amidation reactions of the present invention may be coconut fatty acids, palm oil fatty acids, palm kernel fatty acids or combinations thereof among others. The fatty acid stock may be in treated form or not. Treated herein means distilled, hydrogenated, cut, uncut or combinations thereof. The fatty acid stock used in present invention are commercial products of KLK OLEO company under the brand name of Palmera©.

### MANUEL DISHWASHING DETERGENT

As used herein "manuel dishwashing detergent" refers to the compositions that are employed in manual (i.e. hand) dishwashing for cleaning of dishware.

As used herein "cleaning" means applying to a surface for the purpose of cleaning, and/or disinfecting.

As used herein "dishware" means a surface such as dishes, glasses, pots, pans, baking dishes and flatware made from ceramic, china, metal, glass, plastic (polyethylene, polypropylene, polystyrene, etc.) and wood.

Manuel dishwashing detergent comprises betaine produced by the present invention as an amphoteric surfactant, and comprises other ingredients selected from the groups of anionic surfactants, nonionic surfactants, chelants, hydrotropes, suds stabilizers, perfumes, dyes, pearlescent agents, opacifiers, enzymes, thickening agents, rheology modifiers, preservatives, disinfecting agents, pH tuning means, salts and water. Manual dishwashing detergent may comprise up to 15% wt., preferably 5-10% wt. of betaines produced according to the present invention. The preferred form of the manual dishwashing detergent is liquid.

### ANALYSIS METHODS

**Betaine Content:** The betaine content was determined by means of titration with %67 by weight of perchloroacetic acid in 1,4-dioxane. The solvent used for the betaine was a methanol/ethylene glycol monomethyl ether mixture (2:3), and the electrode used was a pH electrode.

**Water Content:** The water content was determined by using a TitroLine® Alpha Plus Karl Fischer titrator (SI Analytics) with Apura® (Merck) solvent and Apura®5 (Merck) titrant.

**Sodium Chloride Content** :The content of sodium chloride was determined by means of titration with AgNO₃ solution at the presence of K₂CrO₄ indicator.

**Viscosity:** The viscosity was measured at 25°C by using a Brookfield viscometer (LVT) with spindle No. 2 at 12 rpm in a 150 ml sample.

**Acid Value (AV):** The acid value is milligrams of potassium hydroxide required to neutralize the free fatty acids present in 1 g of sample, and is expressed in milligrams per gram. It is a measure of the free acids contained in fats and technical grade fatty acids. The acid values were determined by the standard methods of ISO660:2009(E).

**Gelation in Betaine Solution:** It is determined by visually controlling the presence of gelation in betaine solution obtained from the cooling process of carboxymethylation step. If "gelation in betaine solution" is denoted by "No", this means that any amount of gellified portion was not observed visually in betaine solution obtained from the cooling process of carboxymethylation step. In some cases, it was observed that the gellified portion in betaine solution disappeared in 24h period without any external influence (mixing, heating etc.). At these cases, "gelation in betaine solution" was also denoted as "No".

**Gel Point:** Gel point is the temperature at which no fluidity of the solution at the walls of the glass tube was observed while the betaine solution contained in a glass tube is cooled in a cooling bath of ice-water mixture under mixing.

**Freezing Point:** Freezing point was determined by observing a temperature at which no fluidity of the whole solution contained in a glass tube was reached while the betaine solution in a glass tube was cooled in a cooling bath of ice-water mixture under mixing.

**Cloud Point:** Cloud point is the temperature at which betaine solution starts to phase separation and thus becomes cloudy. It was determined by observing a temperature at which a cloudy appearance occurred while the betaine solution in a glass tube was cooled in a cooling bath of ice-water mixture under mixing.

**Preservation Efficacy Test:** The test consists of challenging the samples with a prescribed inoculum of suitable microorganisms, storing the inoculated preparation at ambient temperature, avoding sunlight, withdrawing the samples from the container at specified intervals of time and counting the organisms in the samples so removed. The preservative properties of the sample are adequate if, in the conditions of the test, there is a significant fall or no increase, as appropriate, in the number of microorganisms in the inoculated preparation after 2, 7, 14 and 28 days.

**Carbon Distribution of fatty acid stock:** Carbon distribution of fatty acid stock was determined by means of GC after derivatization of fatty acids to fatty acid methyl esters.

### EXAMPLES

Exemplary, non-limiting embodiments of the present invention were discussed in detail below to provide a clear understanding.

### EXAMPLE SET 1: EFFECT OF MIXTURE OF FATTY ACID TYPES ON BETAINE CONCENTRATION AND GELATION

**Amidation:** 1:1.05 mole ratio of fatty acid: DMAPA were initially introduced in a 0.5-L five-neck flask whose center neck was equipped with a mechanical stirrer, and two of the side-necks were fitted with a Liebig condenser and a thermocouple. The reaction mixture was brought to 160°C in a silicon oil heating bath and maintained at this temperature for 5,5-6h. The water formed during the reaction was continuously removed from the reaction medium by the Liebig condenser. The obtained product amido amine was left for cooling at room temperature.

**Carboxymethylation:₋** 1:1.05 mole ratio of amidoamine: SMCA and the appropriate amount of water to achieve the targeted betaine concentration were weighed into a 0.5-L five-neck flask fitted with stirrer, thermocouple and reflux condenser. The reaction mixture was then heated to 98°C in a silicone oil heating bath at an average heating rate of 2-3°C/min and the temperature was maintained about 6h under reflux. The flask was then taken out from the heating bath and the obtained product betaine solution was left for cooling at room temperature to 64-72°C. The applied average cooling rates were between 0.73-1.09°C/min. The results of Example Set 1 are given in Table 2.

**Table 2. The Results of Example Set 1 (with high cooling rate)**

| Example # | 1-a | 1-b | 1-c | 1-d | 1-e | 1-f |
|---|---|---|---|---|---|---|
| Targeted betaine concentration (% wt.) | 31.11 | 31.94 | 34.00 | 28.00 | 29.02 | 29.94 |
| Fatty acid stock type | B1210^{∗} | B1210 | B1210 | B1220^{∗∗} | B1220 | B1220 |
| Carboxymethylation cooling rate (°C/min) | 0.91 | 0.97 | 1.09 | 0.73 | 0.87 | 0.84 |
| Carboxymethylation cooling temperature (°C) | 66 | 68 | 72 | 68 | 64 | 66 |
| Cooling medium in carboxymethylation | Ambient air directly in touch with the reaction flask | Ambient air directly in touch with the reaction flask | Ambient air directly in touch with the reaction flask | Ambient air directly in touch with the reaction flask | Ambient air directly in touch with the reaction flask | Ambient air directly in touch with the reaction flask |
| Gelation in betaine solution*** | No | No | Yes | No | No | Yes |
| Analysis: Betaine content (% wt.) | 30.95 | 31.69 | was not determined**** | 27.58 | 28.38 | was not determined |
| Analysis: Sodium chloride (% wt.) | 6.08 | 6.05 | was not determined | 5.4 | 5.49 | was not determined |
| Analysis: Viscosity (cP) | 125 | 125 | was not determined | 25 | 25 | was not determined |
| Analysis: pH | 5.53 | 5.66 | was not determined | 5.88 | 6.12 | was not determined |

| | | | | | | |
|---|---|---|---|---|---|---|
| ^{∗} Palmera© B1210 distilled coconut fatty acid. Carbon distribution by weight % is 5.33 C₈, 6.38 C₁₀, 51.13 C₁₂, 17.66 C₁₄, 7.43 C₁₆, 1.74 C₁₈, 7.63 C_{18:1}, 1.1 C_{18:2}. ^{∗∗} Palmera© B1220 topped palm kernel fatty acid. Carbon distribution by weight % is 0.71 C₁₀, 52.26 C₁₂, 17.32 C₁₄, 9.36 C₁₆, 2.34 C₁₈, 15.40 C_{18:1}, 2.25 C_{18:2}. ^{∗∗∗} as defined in "Analysis Methods" section. ^{∗∗∗∗} The betaine solution was not considered as flowable since gelation occurred. Thus, the related analysis was not done. | | | | | | |

Example 1-b showed that flowable, pumpable, low viscosity aqueous solutions of betaines with up to 32% wt. betaine content in an additive-free process may be obtained if the fatty acid stock used in the amidation step comprises less than 9% (i.e. 8.73%) by weight of total unsaturated C₁₈ fractions and 11-15% (i.e. 11.71%) by weight of C₈ + C₁₀ fractions, and cooling rates applied in carboxymethylation steps are fast (≥0.7°C/min).

Betaine solutions with a betaine content > 32% wt. could not be produced without gelation on the final betaine solution due to high cooling rates as it can be seen in Example 1-c. On the other hand, it was even not possible to produce a betaine solution with 30% wt. betaine content from the fatty acid stock having 17.65% by weight of total unsaturated C₁₈ fraction and 0.71% by weight of C₈ + C₁₀ fraction while the applied cooling were fast (≥0.7°C/min) (see example 1-f).

### EXAMPLE SET 2 : EFFECT OF COOLING RATE OF CARBOXYMETHYLATION STEP ON BETAINE CONCENTRATION AND GELATION

**Amidation:** 1:1.05 mole ratio of fatty acid:DMAPA were initially introduced in a 0.5-L five-neck flask whose center neck was equipped with a mechanical stirrer, and two of the side-necks were fitted with a Liebig condenser and a thermocouple. The reaction mixture was brought to 160°C in a silicon oil heating bath and maintained at this temperature for 5,5-6h. The water formed during the reaction was continuously removed from the reaction medium by the Liebig condenser. The obtained product amidoamine was left for cooling at room temperature.

**Carboxymethylation:₋** 1:1.05 mole ratio of amidoamine:SMCA and the appropriate amount of water to achieve the targeted betaine concentration were weighed into a 0.5-L five-neck flask fitted with stirrer, thermocouple and reflux condenser. The reaction mixture was then heated to 98°C to reflux in a silicone oil heating bath at an average heating rate of 2-3°C/min and the temperature was maintained about 6h under reflux. Having completed the reaction, heating of the silicone oil bath was terminated. The flask was then left in silicon heating bath and the obtained product betaine solution was left for cooling to 57-62°C. The applied average cooling rates were between 0,28-0,36°C/min. The results of Example Set 2 are given in Table 3.

Example 2-b revealed that flowable, pumpable, low viscosity aqueous solutions of betaines with 35% wt. betaine content in an additive-free process can be obtained if the fatty acid stock used in the amidation comprises less than 9% (i.e. 8.73%) by weight of total unsaturated C₁₈ fractions and 11-15% (i.e. 11.71%) by weight of C₈ + C₁₀ fractions provided that the cooling rates applied in carboxymethylation step are about 0.3°C/min.

Note that the only major difference between Example Set 1 and Example Set 2 was cooling rate applied in carboxymethylation reaction. The cooling rate applied in Example Set 1 was fast (≥0.7°C/min) whereas the cooling rate applied in Example Set 2 was slow (≤0.36°C/min). Thus, it was clearly realized that cooling rate applied in carboxymethylation reaction has a crucial effect on the maximum obtainable betaine content of betaine solution without gelation.

By comparing Example 1-a-b-c and Example 2-a-b-c, it can be noticed that switching from fast cooling rate to slow cooling rate it is possible to obtain 3 % weight more concentrated betaine solutions (31.94% to 35%) without gel formation for the fatty acid stock B1210 which comprises less than 9% by weight of total unsaturated C₁₈ fractions and about 12% by weight of C₈ + C₁₀ fractions. By comparing Example 1-d-e-f and Example 2-d-e-f, it can be noticed that switching from fast cooling rate to slow cooling rate it is possible to obtain 2 % wt. more concentrated betaine solutions (29.02% to 30.92%) without gel formation for the fatty acid stock B1220 which has 17.65% by weight of total unsaturated C₁₈ fractions and 0.71% C₈ + C₁₀ fraction.

**Table 3. The results of Example Set 2 (slow cooling rate)**

| Example # | 2-a | 2-b | 2-c | 2-d | 2-e | 2-f |
|---|---|---|---|---|---|---|
| Targeted betaine concentration (% wt.) | 34.00 | 35.00 | 36.07 | 29.94 | 30.92 | 31.96 |
| Fatty acid stock type | B1210* | B1210 | B1210 | B1220** | B1220 | B1220 |
| Carboxymethylation cooling rate (°C/min) | 0.33 | 0.33 | 0.28 | 0.36 | 0.33 | 0.30 |
| Carboxymethylation cooling temperature (°C) | 61 | 57 | 62 | 60 | 57 | 61 |

| Cooling medium in carboxymethylation | Hot silicone oil cooled by ambient air directly in touch with the reaction flask | Hot silicone oil cooled by ambient air directly in touch with the reaction flask | Hot silicone oil cooled by ambient air directly in touch with the reaction flask | Hot silicone oil cooled by ambient air directly in touch with the reaction flask | Hot silicone oil cooled by ambient air directly in touch with the reaction flask | Hot silicone oil cooled by ambient air directly in touch with the reaction flask |
|---|---|---|---|---|---|---|
| Gelation in betaine solution*** | No | No | Yes | No | No | Yes |
| Analysis: Betaine content (% wt.) | 33.91 | 35.00 | was not determined**** | 29.69 | 30.41 | was not determined |
| Analysis: Sodium chloride (% wt.) | 6.54 | 6.74 | was not determined | 6.25 | 6.03 | was not determined |
| Analysis: Water content (% wt.) | 56.4 | 56.02 | was not determined | 55.53 | 59.33 | was not determined |
| Analysis: Viscosity (cP) | 125 | 175 | was not determined | 25 | 75 | was not determined |
| Analysis: pH | 5.74 | 5.63 | was not determined | 6.23 | 6.42 | was not determined |

| | | | | | | |
|---|---|---|---|---|---|---|
| ^{∗} Palmera© B1210 distilled coconut fatty acid. Carbon distribution by weight % is 5.33 C₈, 6.38 C₁₀, 51.13 C₁₂, 17.66 C₁₄, 7.43 C₁₆, 1.74 C₁₈, 7.63 C_{18:1}, 1.1 C_{18:2}. ^{∗∗} Palmera© B1220 topped palm kernel fatty acid. Carbon distribution by weight % is 0.71 C₁₀, 52.26 C₁₂, 17.32 C₁₄, 9.36 C₁₆, 2.34 C₁₈, 15.40 C_{18:1}, 2.25 C_{18:2}. ^{∗∗∗} as defined in "Analysis Methods" section. ^{∗∗∗∗} The betaine solution was not considered as flowable since gelation occurred. Thus, the related analysis was not done. | | | | | | |

### EXAMPLE SET 3 : EFFECT OF ADDING GLYCERINE IN CARBOXYMETHYLATION STEP ON BETAINE CONCENTRATION AND GELATION

**Amidation:** 1:1.05 mole ratio of fatty acid:DMAPA were initially introduced in a 0.5-L five-neck flask whose center neck was equipped with a mechanical stirrer, and two of the side-necks were fitted with a Liebig condenser and a thermocouple. The reaction mixture was brought to 160°C in a silicon oil heating bath and maintained at this temperature for 5,5-6h. The water formed during the reaction was continuously removed from the reaction medium by the Liebig condenser. The obtained product amidoamine was left for cooling at room temperature.

**Carboxymethylation:₋** 1:1.05 mole ratio of amidoamine:SMCA, the appropriate amount of water to achieve the targeted betaine concentration and glycerine %4-10 by weight of final betaine solution were weighed into a 0.5-L five-neck flask fitted with stirrer, thermocouple and reflux condenser. The reaction mixture was then heated to 98°C in a silicone oil heating bath at an average heating rate of 2-3°C and the temperature was maintained about 6h under reflux. Having completed the reaction, heating of the silicone oil bath was terminated. The flask was then left in silicon heating bath and the product (betaine solution) was left for cooling to 59-64°C. The applied average cooling rates were between 0,31-0,34°C/min. The results of Example Set 3 are given in Table 4.

**Table 4. The Results of Example Set 3 (Glycerin addition)**

| Example # | 3-a | 3-b | 3-c | 3-d | 3-e | 3-f |
|---|---|---|---|---|---|---|
| Targeted betaine concentration (% wt.) | 35.00 | 36.07 | 36.07 | 30.92 | 31.96 | 31.96 |
| Fatty acid stock type | B1210* | B1210 | B1210 | B1220** | B1220 | B1220 |
| Carboxymethylation cooling rate (°C/min) | 0.31 | 0.31 | 0.32 | 0.31 | 0.34 | 0.33 |
| Carboxymethylation cooling temperature (°C) | 59 | 59 | 64 | 59 | 59 | 60 |
| Cooling medium in carboxymethylation | Hot silicone oil cooled by ambient air directly in touch with the reaction flask | Hot silicone oil cooled by ambient air directly in touch with the reaction flask | Hot silicone oil cooled by ambient air directly in touch with the reaction flask | Hot silicone oil cooled by ambient air directly in touch with the reaction flask | Hot silicone oil cooled by ambient air directly in touch with the reaction flask | Hot silicone oil cooled by ambient air directly in touch with the reaction flask |
| Glycerine addition in carboxymethylation | 4% by wt. of final betaine solution | 4% by wt. of final betaine solution | 10% by wt. of final betaine solution | 4% by wt. of final betaine solution | 4% by wt. of final betaine solution | 10% by wt. of final betaine solution |
| Gelation in betaine solution*** | No | Yes | Yes | No | Yes | Yes |
| Analysis: Betaine content (% wt.) | 34.65 | was not determined**** | was not determined | 30.31 | was not determined | was not determined |
| Analysis: Sodium chloride (% wt.) | 6.95 | was not determined | was not determined | 5.62 | was not determined | was not determined |
| Analysis: Water content (% wt.) | 52.52 | was not determined | was not determined | 59.86 | was not determined | was not determined |
| Analysis: Viscosity (cP) | 300 | was not determined | was not determined | 75 | was not determined | was not determined |
| Analysis: pH | 5.79 | was not determined | was not determined | 6.6 | was not determined | was not determined |

| | | | | | | |
|---|---|---|---|---|---|---|
| ^{∗} Palmera© B1210 distilled coconut fatty acid. Carbon distribution by weight % is 5.33 C₈, 6.38 C₁₀, 51.13 C₁₂, 17.66 C₁₄, 7.43 C₁₈, 1.74 C₁₈, 7.63 C_{18:1}, 1.1 C_{18:2}. ^{∗∗} Palmera© B1220 topped palm kernel fatty acid. Carbon distribution by weight % is 0.71 C₁₀, 52.26 C₁₂, 17.32 C₁₄, 9.36 C₁₆, 2.34 C₁₈, 15.40 C_{18:1}, 2.25 C_{18:2}. ^{∗∗∗} as defined in "Analysis Methods" section. ^{∗∗∗∗} The betaine solution was not considered as flowable since gelation occurred. Thus, the related analysis was not done. | | | | | | |

Example 3-a revealed that flowable, pumpable aqueous solutions of betaines with 35% wt. betaine content may be obtained if the fatty acid stock used in the amidation step comprises less than 9% (i.e. 8.73%) by weight of total unsaturated C₁₈ fractions and 11-15% (i.e. 11.71%) by weight of C₈ + C₁₀ fractions, and the cooling rate applied in carboxymethylation step is about 0.3°C/min, and glycerine 4% by weight of final betaine solution is added before the carboxymethylation reaction.

Note that the only difference between the processes of Example 2-b and Example 3-a was glycerine addition before carboxymethylation reaction. Example 3-a included glycerine addition but Example 2-b did not. It is also noteworthy to say that glycerine addition caused higher viscosity in final betaine solution compared to the betaine solutions obtained by glycerine-free process.

### EXAMPLE SET 4: EFFECT OF ADDING TRIMETHYLAMINE (TMA) IN CARBOXYMETHYLATION STEP ON BETAINE CONCENTRATION AND GELATION

**Amidation:** 1:1.05 mole ratio of fatty acid:DMAPA were initially introduced in a 0.5-L five-neck flask whose center neck was equipped with a mechanical stirrer, and two of the side-necks were fitted with a Liebig condenser and a thermocouple. The reaction mixture was brought to 160°C in a silicon oil heating bath and maintained at this temperature for 5,5-6h. The water formed during the reaction was continuously removed from the reaction medium by the Liebig condenser. The obtained product amidoamine was left for cooling at room temperature.

**Carboxymethylation:₋** 1:1.05 mole ratio of (amidoamine+TMA): SMCA, the appropriate amount of water to achieve the targeted betaine concentration were weighed into a 0.5-L five-neck flask fitted with stirrer, thermocouple and reflux condenser. The reaction mixture was then heated to 98°C in a silicone oil heating bath at an average heating rate of 2-3°C/min and the temperature was maintained about 6h under reflux. Having completed the reaction, heating of the silicone oil bath was terminated. The flask was then left in silicon heating bath and the obtained product betaine solution was left for cooling to 58-61°C. The applied average cooling rates were between 0,29 -0,36°C/min. The results of Example Set 4 are given in Table 5.

Example 4-a revealed that flowable, pumpable, low-viscosity aqueous solutions of betaines with 35% wt. betaine content may be obtained if the fatty acid stock used in the amidation comprises less than 9% (i.e. 8.73%) by weight of total unsaturated C₁₈ fractions and 11-15% (i.e. 11.71%) by weight of C₈ + C₁₀ fractions, and the cooling rate applied in carboxymethylation step is 0,36°C/min, and TMA is added before the carboxymethylation reaction.

Comparing Example 4-a and Example 2-b, it was clearly observed that TMA addition before carboxymethylation reaction caused a significant decrease in viscosity (175 cP to 62.5 cP) and in pH (5.63 to 3.83) of final betaine solution.

**Table 5. The results of Example Set 4 (TMA addition)**

| Example # | 4-a | 4-b | 4-c | 4-d | 4-e |
|---|---|---|---|---|---|
| Targeted betaine concentration (% wt.) | 34.60^{a} | 35.40^{a} | 30.60^{a} | 32.55^{a} | 33.30^{a} |
| Fatty acid stock type | B1210* | B1210 | B1220** | B1220 | B1220 |
| Carboxymethylation cooling rate (°C/min) | 0.36 | 0.29 | 0.35 | 0,32 | 0,31 |
| Carboxymethylation cooling temp. (°C) | 58 | 60 | 59 | 59 | 61 |
| Cooling medium in carboxymethylation | Hot silicone oil cooled by ambient air directly in touch with the reaction flask | Hot silicone oil cooled by ambient air directly in touch with the reaction flask | Hot silicone oil cooled by ambient air directly in touch with the reaction flask | Hot silicone oil cooled by ambient air directly In touch with the reaction flask | Hot silicone oil cooled by ambient air directly in touch with the reaction flask |
| TMA addition in carboxymethylation | 10:90 TMA:AA∗∗∗ mole ratio | 10:100 TMA:AA mole ratio | 10:90 TMA:AA mole ratio | 10:100 TMA:AAmole ratio | 10:100 TMA:AA mole ratio |
| Gelation in betaine solution**** | No | Yes | No | No | Yes |
| Analysis: Betaine content (% wt.) | 34.85 | was not determined***** | 30.55 | 31.83 | was not determined |
| Analysis: Sodium chloride (% wt.) | 7.30 | was not determined | 6.31 | 6.58 | was not determined |
| Analysis: Water content (% wt.) | 61.02 | was not determined | 68.45 | 63.49 | was not determined |
| Analysis: Viscosity (cP) | 62.50 | was not determined | 62.50 | 150 | was not determined |
| Analysis: pH | 3.83 | was not determined | 4.03 | 4.53 | was not determined |

| | | | | | |
|---|---|---|---|---|---|
| ^{a} Targeted betaine content includes both carboxymethylated amidoamine and carboxymethylated TMA ^{∗} Palmera© B1210 distilled coconut fatty acid. Carbon distribution by weight % is 5.33 C₈, 6.38 C₁₀, 51.13 C₁₂, 17.66 C₁₄, 7.43 C₁₆, 1.74 C₁₈, 7.63 C_{18:1}, 1.1 C_{18:2}. ^{∗∗} Palmera© B1220 topped palm kernel fatty acid. Carbon distribution by weight % is 0.71 C₁₀, 52.26 C₁₂, 17.32 C₁₄, 9.36 C₁₆, 2.34 C₁₈, 15.40 C_{18:1}, 2.25 C_{18:2}. ^{∗∗∗} AA=amidoamine ^{∗∗∗∗} as defined in "Analysis Methods" section. ^{∗∗∗∗∗} The betaine solution was not considered as flowable since gelation occurred. Thus, the related analysis was not done. | | | | | |

### EXAMPLE SET 5: PILOT SCALE ADDITIVE-FREE BETAINE PRODUCTION TRIALS

**Amidation:** 1:1.05 mole ratio of fatty acid: DMAPA were initially introduced into a 30-L jacketed and stirred stainless steel pilot reactor equipped with a vertical heat exchanger followed by a horizontal heat exchanger on the top of the reactor where outlet stream of horizontal heat exchanger can be refluxed to the reactor or can be removed from the system and stored in a vessel if desired. The reaction mixture was brought to 160°C by circulating hot heat-transfer oil in the jacket of the reactor. The reaction was maintained at this temperature for 8h. The water formed during the reaction was continuously removed from the reactor. The obtained product amidoamine was cooled by the said heat-transfer oil after cooling the said heat-transfer oil with cooling water in another heat exchanger and circulating it through the jacket of the reactor.

**Carboxymethytation:₋** 1:1.05 mole ratio of amidoamine:SMCA, the appropriate amount of water to achieve the targeted betaine concentration were charged into the same pilot reactor. The reaction mixture was then heated to 98°C by circulating hot heat-transfer oil in the jacket of the reactor. The average heating rate was 2.04°C. The temperature was maintained for about 6h under reflux. Having completed the reaction, heating of the heat-transfer oil was terminated. The obtained product betaine solution was cooled to 60°C by the said heat-transfer oil. At the cooling stage, the hot heat-transfer oil was not cooled by cooling water but was cooled by the ambient air as it circulated through the system. The obtained average cooling rate was 0.26°C/min. The results of Example Set 5 are given in Table 6.

**Table 6. The results of Example Set 5 (Pilot scale reaction without additive)**

| Example # | 5-a | 5-b |
|---|---|---|
| Targeted betaine concentration (% wt.) | 35.22 | 35.19 |
| Fatty acid stock type | B1210-2* | B1213** |
| Carboxymethylation cooling rate (°C/min) | 0.26 | 0.27 |
| Carboxymethylation cooling temp. (°C) | 60 | 60 |
| Cooling medium in carboxymethylation | Hot heat-transfer oil circulates through the jacket of the reactor as it is cooled by the ambient air | Hot heat-transfer oil circulates through the jacket of the reactor as it is cooled by the ambient air |
| Gelation in betaine solution*** | No | No |
| Analysis: Betaine content (% wt.) | 35.38 | 34.14 |
| Analysis: Sodium chloride (% wt.) | 6.63 | 6.56 |
| Analysis: Water content (% wt.) | 53.43 | 54.53 |
| Analysis: Viscosity (cP) | 112.5 | 87.5 |
| Analysis: pH | 6.62 | 7.40 |
| Freezing point | <-6°C | 10°C |
| Gel point | <-6°C | 14°C |
| Cloud point | <-6°C | 15°C |

| | | |
|---|---|---|
| ^{∗} Palmera© B1210 distilled coconut fatty acid. Carbon distribution by weight % is 6.80 C₈, 7.84 C₁₀, 51.44 C₁₂, 17.11 C₁₄, 6.89 C₁₆, 1.09 C₁₈, 7.60 C_{18:1}, 1.24 C_{18:2}. ^{∗∗} Palmera© B1213 distilled hydrogenated coconut fatty acid. Carbon distribution by weight % is 5.61 C₈, 8.59 C₁₀, 49.54 C₁₂, 17.75 C₁₄, 8.28 C₁₆, 8.41 C₁₈. ^{∗∗∗} as defined in "Analysis Methods" section. | | |

Example 5-a revealed that flowable, pumpable, low-viscosity aqueous solutions of betaines with 35% wt. betaine content in an additive-free process carried out in a pilot reactor may be obtained if the fatty acid stock used in the amidation step comprises less than 9% (i.e. 8.84%) by weight of total unsaturated C₁₈ fractions and 11-15% (i.e. 14.64%) by weight of C₈ + C₁₀ fractions provided that the cooling rate applied in carboxymethylation step is about 0.26°C/min.

Note that, fatty acid stock which did not include unsaturated C₁₈ fractions (B1213, Example 5-b) did also yield up to 34-35% wt. betaine containing solutions, however, freezing, gelation and cloud points of this solution were significantly higher than that of betaine solution obtained from the fatty acid stock comprising total unsaturated C₁₈ fractions about 9% by weight (B1210-2, Example 5-a). Thus, the betaine solution of Example 5-b was not considered flowable and pumpable at low temperatures (-5 - 20°C).

### Preservation Efficacy Test Results

The preservation efficacy tests of Example 2-b and Example 5-a, carried out according to test method described i n Analysis Method section, are given in Table 7 and Table 8, respectively.

**Table 7. The Preservation Efficacy Test Results of Example2-b**

| | Inoculation | 0 time | 2^{nd} day | 7^{th} day | 14^{th} day | 28^{th} day |
|---|---|---|---|---|---|---|
| Pseudomonas aeruginosa | 6.8E+05 | 4.1E+05 | <100 | <10 | <10 | <10 |
| Staphylococcus aureus | 2.7E+05 | 4.5E+05 | <100 | <10 | <10 | <10 |
| E. coli | 5.2E+05 | 5.2E+05 | <100 | <10 | <10 | <10 |
| Candida albicans | 5.0E+05 | 4.5E+05 | <100 | <10 | <10 | <10 |
| Aspergilus brasillienss | 3.8E+04 | 3.7E+04 | <100 | <10 | <10 | <10 |

| | | | | | | |
|---|---|---|---|---|---|---|
| Units in cfu/g | | | | | | |

**Table 8. The Preservation Efficacy Test Results of Example 5-a**

| | Inoculation | 0 time | 2^{nd} day | 7^{th} day | 14^{th} day | 28^{th} day |
|---|---|---|---|---|---|---|
| Pseudomonas aeruginosa | 6.8E+05 | 3.4E+05 | <100 | <10 | <10 | <10 |
| Staphylococcus aureus | 5.4E+05 | 5.9E+05 | <100 | <10 | <10 | <10 |
| E. coli | 5.2E+05 | 4.6E+05 | <100 | <10 | <10 | <10 |
| Candida albicans | 5.0E+05 | 3.2E+05 | <100 | <10 | <10 | <10 |
| Aspergilus brasillienss | 3.8E+04 | 3.5E+04 | <100 | <10 | <10 | <10 |

| | | | | | | |
|---|---|---|---|---|---|---|
| Units in cfu/g | | | | | | |

As it can be seen from Table 7 and Table 8, flowable, pumpable, low-viscosity betaine solutions (35% wt. betaine content) produced accordingly by the additive-free process of present invention demonstrates adequate results in preventing microorganism growth , since inoculated microorganisms decreased or remained constant during test period, although in the present invention used betaine solutions did not comprise any preservatives.

### MANUAL DISHWASHING DETERGENT EXAMPLE

By using the betaine solution produced according to the present invention, the following liquid manual dishwashing detergent formulation was prepared as a non-limiting example.

| | %, wt. |
|---|---|
| Sodium lauryl ether sulphate - 2EO (Anionic surfactant, TEXAPON N-70 from BASF, 70% wt.) | 10.00 |
| Cocamidopropyl betaine (Amphoteric surfactant, produced accordingly to present Invention - Example 5-a, 35% wt.) | 5.73 |
| OPACIFIER 301 E (Opacifier) | 0.30 |
| PARMETOL A - 28 S (Preservative) | 0.10 |
| Magnesium sulphate heptahydrate (thickening agent, 100% wt.) | 2.00 |
| Citric acid (pH tuning) | 0.05 |
| Perfume | 0.23 |
| De-ionized water (as required to complete 100%) | 81.59 |
| TOTAL | 100.00 |

### MANUEL DISHWASHING DETERGENT PERFORMANCE TEST (FOAMING TEST)

**Comparative Example** : The above Manuel Dishwashing Detergent formulation was also prepared for comparison purposes by replacing the betaine solution with a commercial betaine solution in that the betaine contents were set equal and the rest of the ingredients were kept at the exact same amounts.

**Foaming Tests Apparature:** The foaming tests are performed with an internally designed test equipment comprising a rotating motor and cylinders having 4 cm of inner diameter and 70 cm of height. Cylinders are rotated vertically.

**Foam Height Test** :Samples were provided by preparing a solution of 0.5 g formulation in 2-L of water which has 20°Fr hardness. 200 ml of the prepared samples were put in cylinders stoppered and mixed by rotating the cylinders vertically for 3 minutes with 40 revolution per minute, stopped and the foam height was measured .

**Foam Height Test in Presence of Oil :** Samples were provided by preparing a solution of 0.5 g formulation in 2-L of water which has 20°Fr hardness. 200 ml of the prepared samples and 4 drops of sun flower oil were put in cylinders stoppered and mixed by rotating the cylinders vertically for 2 minutes with 40 revolution per minute, stopped and the foam height was measured .

The sun flower oil load and 2-min mixing period was repeated until the foam dissappeared.

**Foaming Test Results:**

| | Formulation with betaine solution prepared according to the inventive process (Example 5-a) | Formulation with commercial betaine solution (Comparative Example) |
|---|---|---|
| Foam height (mm) (without oil load) | 148 | 150 |
| Foam height (mm) (4 drops oil load) | 20 | 20 |
| Foam height (mm) (8 drops oil load) | 10 | 10 |

All of the results are average of 4 trials.

The performed Foaming Tests clearly indicates that formulations containing the betaine solution (35% wt. betaine content) produced by the present invention showed an equal foaming performance with a formulation containing a betaine solution commercially available in the market.

## Claims

1. A process for the preparation of gelling free, flowable and pumpable aqueous betaine solution with a betaine content of ≤ % 35 by weight having the general formula (I), 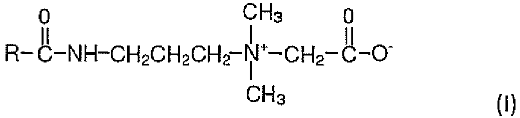 in which R is a mixture of C₈-C₁₈ alkyl and C₈-C₁₈ unsaturated alkyl radical the said process comprising
(i) reacting a fatty acid stock comprising 11-15 wt. % C₈+C₁₀ fractions and less than 9% wt. of total unsaturated C₁₈ fractions with 3-dimethylaminopropylamine in solvent free environment by removing of during the reaction formed water to yield amidoamine compound and
(ii) reacting in step (i)obtained amidoamine reaction mixture with sodium monochloroacetate or monochloroacetic acid in the presence of sodium hydroxide in an aqueous medium to yield a betaine solution
(iii) applying a gradual cooling process to the in step (ii) obtained betaine solution by use of a cooling medium
wherein in (iii) said gradual cooling process is carried out with a cooling rate which is below 0.5°C/min and wherein in step (iii) said cooling process is carried out until the said betaine solution is cooled from 80-100°C, which is the temperature at which step (ii) takes place, to at least 70°C.

2. A process according to claim 1, wherein said process is carried out additive free.

3. A process according to claim 1, wherein in step (ii) trimethylamine is added in 10:60 to 60:100 by mole ratio of trimethylamine:amidoamine.

4. A process according to claim 1, wherein in step (ii) glycerine is added up to 4% by weight.

5. A process according to any preceding claim, wherein in step (i) said fatty acid stock is distilled coconut fatty acid.

6. A process according to claim 5, wherein the total unsaturated C₁₈ fractions comprises C_{18:1} and C_{18:2} between a 75:25 - 95:05, preferably 85:15-90:10 ratio by weight.

7. The process according to claim 5, wherein the C₈:C₁₀ ratio by weight is between 40:60 - 60:40, preferably 45:55-55:45 by weight.

8. A process according to any preceding claim, wherein in step (iii) said cooling process is carried out until the said betaine solution is cooled to 65°C, preferably 60°C.

9. A process according to any preceding claim wherein said cooling process is carried out until the said betaine solution is cooled to at least 60°C.

10. A process according to any preceding claim, wherein in step (iii) said cooling medium is heat-transfer oil.

## Patentansprüche

1. Verfahren zur Herstellung von gelierfreiem, fließfähige und pumpbare wässrige Betainlösung mit einem Betain-Gehalt von ≤ 35 Gew.-% mit der allgemeinen Formel (I), 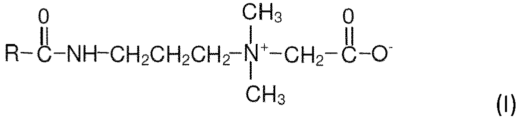 in welchem R ist eine Mischung aus C₈-C₁₈-Alkyl und C₈-C₁₈-ungesättigtem Alkylradikal, wobei das Verfahren umfasst
(i) Umsetzen eines Fettsäurevorrats, der 11-15 Gew.-% C₈+C₁₀-Fraktionen und weniger als 9 Gew.-% der gesamten ungesättigten C₁₈-Fraktionen umfasst, mit 3-Dimethylaminopropylamin in lösungsmittelfreier Umgebung durch Entfernen von während der Reaktion gebildetem Wasser, um eine Amidoaminverbindung zu ergeben, und
(ii) Umsetzen der in Schritt (i) erhaltenen Amidoamin-Reaktionsmischung mit Natriummonochloracetat oder Monochloressigsäure in Gegenwart von Natriumhydroxid in einem wässrigen Medium, um eine Betainlösung zu erhalten
(iii) Anwenden eines allmählichen Abkühlungsprozesses auf den in Schritt (ii) erhaltene Betainlösung unter Verwendung eines Kühlmediums
**dadurch gekennzeichnet, dass** in (iii) der allmähliche Abkühlungsprozess wird durchgeführt mit einer Abkühlgeschwindigkeit, die unter 0.5°C/min liegt und dass in Schritt (iii) der Abkühlungsprozess wird durchgeführt bis die Betainlösung von 80-100°C abgekühlt ist, dies ist die Temperatur, bei der Schritt (ii) stattfindet, auf mindestens 70°C.

2. Verfahren nach Anspruch 1, **dadurch gekennzeichnet, dass** das Verfahren additivfrei durchgeführt wird.

3. Verfahren nach Anspruch 1, **dadurch gekennzeichnet, dass** in Schritt (ii) Trimethylamin in einem Molverhältnis von 10:60 bis 60:100 von Trimethylamin:Amidoamin zugegeben wird.

4. Verfahren nach Anspruch 1, **dadurch gekennzeichnet, dass** in Schritt (ii) Glycerin bis zu 4 Gew.-% zugesetzt wird.

5. Verfahren nach einem der vorhergehenden Ansprüchen, **dadurch gekennzeichnet, dass** in Schritt (i) der Fettsäurevorrat destillierte Kokosfettsäure ist.

6. Verfahren nach Anspruch 5, **dadurch gekennzeichnet, dass** die gesamten ungesättigten C₁₈-Fraktionen umfassen C_{18:1} und C_{18:2} zwischen einem Gewichtsverhältnis von 75:25-95:05, vorzugsweise 85:15-90:10.

7. Verfahren nach Anspruch 5, **dadurch gekennzeichnet, dass** das C8:C10-Verhältnis zwischen 40:60 - 60:40, vorzugsweise 45:55 - 55:45 nach Gewicht liegt.

8. Verfahren nach einem der vorhergehenden Ansprüchen, **dadurch gekennzeichnet, dass** in Schritt (iii) der Abkühlungsprozess durchgeführt wird, bis die Betainlösung auf 65°C, vorzugsweise 60°C, abgekühlt ist.

9. Verfahren nach einem der vorhergehenden Ansprüchen, **dadurch gekennzeichnet, dass** der Abkühlungsprozess durchgeführt wird, bis die Betainlösung auf mindestens 60°C abgekühlt ist.

10. Verfahren nach einem der vorhergehenden Ansprüchen, **dadurch gekennzeichnet, dass** in Schritt (iii) das besagte Abkühlmedium ist Wärmeträgeröl.

## Revendications

1. Procédé de préparation d'une solution aqueuse de bétaïne exempte de gel, fluide et pompable, dont la teneur en bétaïne est ≤ % 35 en poids, présentant la formule générale (I), 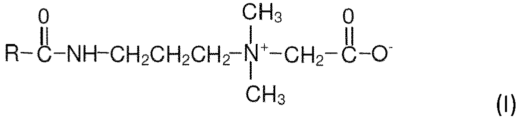 dans laquelle R est un mélange de radical alkyle en C₈-C₁₈ et de radical alkyle insaturé en C₈-C₁₈, ledit procédé comprenant les étapes consistant:
(i) la réaction du matériau acide gras comprenant 11 à 15 % en poids de fractions en C₈+C₁₀ et moins de 9 % en poids de fractions insaturées totales en C₁₈ avec de la 3-diméthylaminopropylamine dans un environnement sans solvant, en éliminant l'eau formée au cours de la réaction pour obtenir un composé amidoamine, et
(ii) la réaction, dans l'étape (i), du mélange réactionnel amidoamine obtenu avec le monochloroacétate de sodium ou l'acide monochloroacétique en présence d'hydroxyde de sodium dans un milieu aqueux pour obtenir une solution de bétaïne
(iii) l'application d'un processus de refroidissement progressif à la solution de bétaïne obtenue à l'étape (ii) en utilisant un milieu de refroidissement,
dans lequel, lors de l'étape (iii), ledit processus de refroidissement progressif s'effectue à une vitesse de refroidissement qui est inférieure à 0,5°C/min et dans lequel, lors de l'étape (iii), ledit processus de refroidissement s'effectue jusqu'à ce que ladite solution de bétaïne soit refroidie de 80-100°C, qui est la température à laquelle l'étape (ii) a lieu, à au moins 70°C.

2. Procédé selon la revendication 1, dans lequel ledit procédé est mené sans additif.

3. Procédé selon la revendication 1, dans lequel, lors de l'étape (ii), la triméthylamine est ajoutée dans un rapport molaire de 10:60 à 60:100 de triméthylamine:amidoamine.

4. Procédé selon la revendication 1, dans lequel, lors de l'étape (ii), la glycérine est ajoutée jusqu'à 4% en poids.

5. Procédé selon l'une quelconque des revendications précédentes, dans lequel, lors de l'étape (i), ledit matériau d'acide gras est un acide gras de noix de coco distillé.

6. Procédé selon la revendication 5, dans lequel les fractions en C₁₈ insaturées totales comprennent des C_{18:1} et des C_{18:2} entre un rapport en poids de 75:25 à 95:05, de préférence 85:15 à 90:10.

7. Procédé selon la revendication 5, dans lequel le rapport en poids C₈:C₁₀ est compris entre 40:60 et 60:40, de préférence 45:55 et 55:45 en poids.

8. Procédé selon l'une quelconque des revendications précédentes, dans lequel, lors de l'étape (iii), ledit processus de refroidissement est mené jusqu'à ce que ladite solution de bétaïne soit refroidie à 65°C, de préférence à 60°C.

9. Procédé selon l'une quelconque des revendications précédentes, dans lequel ledit processus de refroidissement est mené jusqu'à ce que ladite solution de bétaïne soit refroidie à au moins 60°C.

10. Procédé selon l'une quelconque des revendications précédentes, dans lequel, lors de l'étape (iii), ledit milieu de refroidissement est une huile de transfert thermique.
